# EUROPEAN PATENT SPECIFICATION

(11) **EP 4 248 860 B1**
(45) Date of publication and mention of the grant of the patent: **04.11.2026**
(21) Application number: 22170772.2
(22) Date of filing: 29.04.2022
(51) Int. Cl.: A61B 5/145, A61M 5/142

(54) **INSERTION DEVICE AND INSERTION SYSTEM FOR INSERTING A MEDICAL DEVICE**
EINFÜHRVORRICHTUNG UND EINFÜHRSYSTEM ZUM EINFÜHREN EINER MEDIZINISCHEN VORRICHTUNG
DISPOSITIF D'INSERTION ET SYSTÈME D'INSERTION POUR L'INSERTION D'UN DISPOSITIF MÉDICAL

(30) Priority: 24.03.2022 US 202263323190 P
(43) Date of publication of application: 27.09.2023
(73) Proprietor: Roche Diabetes Care GmbH, 68305 Mannheim (DE); F. Hoffmann-La Roche AG, 4070 Basel (CH)
(72) Inventor: CADIO, Michel, 68305 Mannheim (DE)
(74) Representative: Grünecker Patent- und Rechtsanwälte PartG mbB

(56) References cited:
- WO-A1-2021/046255
- US-A1- 2011 319 729
- US-A1- 2020 337 608

## Description

The present invention refers to an insertion device for at least partially inserting a medical device such as an analyte sensor.

Document US20190350527 discloses an insertion device in which a needle carrier is twisted during the retraction process.

Document US9522225B2 describes a needle inserter device in which the insertion process requires the application of force against a spring which provides the force to retract a needle.

Further inserter devices according to the state of the art are for instance described in US2011/0319729A1, US2020/0337608A1 and WO2021/046255A1.

### Summary of the invention

According to the invention, there is provided an insertion device according to claim 1 for at least partially inserting a medical device including an analyte sensor through the skin of a subject, preferred embodiments being specified in the dependent claims 2-13.

### Summary of the disclosure

The object of the present disclosure is to provide an insertion device which is easy to use and avoids rotation of parts that contact a needle which may rotate the needle as such a rotation may affect user comfort.

The insertion device is provided in order to at least partially insert a medical device. This medical device may be, or comprise, an analyte sensor such as a glucose sensor.

At least part of the medical device is to be inserted through the skin of a subject. The subject may be human being such as a patient suffering from diabetes or an animal.

The medical device may be provided for various purposes. It may be provided in order to withdraw samples from the subject e.g. by providing a cannula, it may be provided in order to provide medication or liquids to the subject (also possibly by a cannula) and/or it may be provided to gain information on the subject by sensors such as an analyte sensor like a glucose sensor.

The insertion device has a proximal end that is to be put in contact with the skin and it has a distal end opposite to the proximal end. The device may be arranged such that the user can act on the distal end of the insertion device in order to insert the medical device, while the proximal end is in contact with the skin of the subject, which may be the user or another person or animal.

The size of the insertion device from the distal end to the proximal end may be in the range of 60 to 80 mm such as between 65 and 75 mm. This refers to the size of the insertion device prior to being used for an insertion process. In that state an elastic element is for example still pre-loaded, as will be explained further below and/or no force is applied in order to push the cap and/or the guide sleeve towards each other.

This size of the insertion device from the distal end to the proximal end may depend on the size of the medical device. A larger/smaller medical device may have a larger/smaller insertion device. It may also depend on the length of the portion to be retracted or the desired distance between the end of the portion to be retracted from the skin. The smaller the desired distance the smaller the size of the insertion device.

The insertion device comprises a guide sleeve, which is provided at the proximal end of the insertion device and is to be placed in contact with the skin of the subject for the insertion process. This guide sleeve may exert force on the skin of the user in the insertion process which fixates the guide sleeve on the skin and avoids relative movement of the guide sleeve parallel to the surface of the skin in order to allow a safe insertion process.

A cap is provided at the distal end of the insertion device which may be pressed on to carry out at least part of the insertion process, while moving in the direction of the proximal end of the insertion device. The insertion process may also involve a movement of the cap in the opposite direction from the proximal end of the insertion device towards the distal end of the insertion device. This movement may be shorter than the movement in the direction of the distal end towards the proximal end.

The guide sleeve and the cap together may provide a housing of the insertion device and may be the only visible parts or touchable parts by a human user when the insertion device is provided on the skin of a subject.

The insertion device furthermore comprises an inserter sleeve provided at least partly within the guide sleeve and/or the cap and is provided with a holder for the medical device. The inserter sleeve is provided moveable with respect to the guide sleeve. The inserter sleeve may be adapted to hold the medical device, part of which is to be inserted, and the movement of the inserter sleeve, with respect to the guide sleeve, allows approximating the medical device towards and (partially) through the skin of the user.

The medical device may comprise a part which is to be inserted through the skin of the subject and it may comprise another part which is to be applied on the outside of the skin of the subject e.g.by an adhesive.

The medical device may, for example, be an analyte sensor which is combined with electronics. This sensor is to be inserted through the skin of the subject with e.g. electrodes that can measure an analyte concentration such as a glucose concentration in an interstitial fluid of the subject. The medical device may, furthermore, include a (wearable or portable) electronic circuit which may be battery powered that processes information obtained by the inserted part of the medical device such as by the sensor. The electronics may be configured to be attached to the skin of the subject by an adhesive. The adhesive may be provided on an outer surface of a housing that contains the electronics.

The insertion device, furthermore, comprises a retractor which is configured to retract a portion provided with the medical device such as a needle and the retractor is provided moveable along a movement path. A medical device which has a part that is to be inserted, often is inserted with the use of a portion provided with the medical device such as a needle, which itself is only provided for the insertion process and retracted afterwards. The portion provided with the medical device will not be provided with the medical device after the insertion process. It will be disconnected therefrom. The portion provided with the medical device may give stability to the part/sensor that is to be inserted, which itself may not necessarily be rigid for user comfort. After the part/sensor being inserted, the portion/needle can be retracted and the part/sensor remains inserted.

The insertion device, furthermore, comprises an elastic element such a spring, which is provided to bias the retractor towards the distal end along the movement path.

In order to control the movement of the retractor, a retractor control element is provided which may block or free the movement of the retractor along the movement path. The retractor control element itself is moveable with respect to the retractor. In case the retractor control element frees the movement path of the retractor, the elastic element may move the retractor along this freed movement path. Thereby, a rapid retraction of the portion of the medical device, such as a needle to be retracted, is achieved. The movement of the retractor - once freed to move along the movement path - may include a movement beyond the end of a movement path defined by guide portions of the inserter device.

With such a configuration, it is possible to provide an insertion device with a pre-loaded elastic element such that for the insertion process no force against the elastic element, which may provide the movement of the retractor towards the distal end, is necessary.

Furthermore, as the retractor control element is moveable, with respect to the retractor, the motion of the retractor can be confined to the linear movement, avoiding possible rotational movement of the portion that is to be retracted and thereby on the part that is to be inserted. Avoiding rotational movement of the portion to be retracted or the part to be inserted improves the user experience by avoiding a potential source of pain and by avoiding undesired deformation of the part to be inserted such as of the sensor.

The retractor control element may be rotatable into a position in which it blocks the movement of the retractor along the movement path and another position in which it frees the movement of the retractor along the movement path. The use of a rotational movement of the retractor control element allows the device (e.g. extension of the device in a direction perpendicular to the surface of the skin to which it may be applied, i.e. the size measured from the distal to the proximal end of the insertion device) to be relatively small as, for the rotational movement, no additional space for a longitudinal movement inside the insertion device is necessary.

The retractor control element may be provided rotatable by a portion of the guide sleeve which comes into contact with the retractor control element, such as with a protruding portion of the retractor control element upon a relative movement of the retractor control element, with respect to the guide sleeve. As the relative movement of the retractor element, with respect to the guide sleeve, at least to some extent, is a linear movement the portion of the guide sleeve may be provided with a ramp shape, which translates the linear movement into a rotational movement of the retractor control element, as the protruding portion of the retractor control element slides along the portion of the guide sleeve, such as the ramp which has the ramp shape. This allows for a well-defined rotational movement of the retractor control element.

The movement of the retractor is confined to a linear movement in the direction of the proximal end to the distal end, or vice versa. This confinement to a linear movement avoids any rotational movement of the retractor which may cause a rotation of the part that is to be inserted, or that is inserted into the skin of the subject. The confinement to the linear movement is optionally provided by at least one pair of guide portions, the pair of guide portions may comprise of the first guide portion, such as a rail, and co-operate with a second guide portion such as a negative rail, where one of the two guide portions is provided at the inserter sleeve and the other guide portion is provided at the retractor. The guide portions may be provided as straight elements providing a linear guiding. Such guide portions allow for a safe way to define the linear movement of the retractor. In order to provide a stable and wiggle free movement, optionally, there are three such pairs of guide portions provided. They may be provided with the same distance to each other, or at least two of the three pairs may be provided with the same distance with respect to each other. Thereby, unintentional mechanical blocking of the retractor can well be avoided.

The guide portions of the inserter sleeve may provide a portion of the movement path of the retractor. Another portion of the movement path of the retractor may be provided by guide portions of the retractor control element. The movement path of the retractor may be provided by the guide portion of the inserter sleeve and the guide portions of the retractor control element in combination.

The retractor control element may be provided with at least one guide portion which can be brought into alignment with another guide portion such as the above mentioned guide portion of the inserter sleeve in order to free the movement of the retractor along a portion of the movement path and it may block the movement of the retractor along the portion of the movement path in case the guide portion of the retractor control element is not in such alignment. Using the guide portions that define the linear movement of the retractor, also for mechanically allowing or blocking the movement of the retractor along its movement path, provides for a mechanically simple construction allowing for a failsafe design. The movement path may be provided by aligned guide portions of the inserter sleeve and of the retractor control element.

The retractor control element may be provided with at least one blocking protrusion which is provided to interact with the blocking portion of the cap, preventing unintentional rotation of the retractor control element into a position in which it frees the movement of the retractor. This interaction between a blocking protrusion and a blocking portion of the cap allows to well define the moment in which the retractor control element is allowed to free the path of the retractor. Optionally, this interaction between the blocking protrusion and the blocking portion occur when the cap is completely pressed down i.e. when the cap has reached its most proximal position relative to the skin of the subject. Upon slightly retracting the cap away from this most proximal position towards a more distal position, the interaction may stop, therefore freeing the rotation of the retractor control element towards a position in which it frees the movement of the retractor along the movement path.

With help of this blocking protrusion and the blocking portion it is possible to have the retraction process only to take place once the cap is moved away from the skin of the subject. This provides control to the user of the insertion device in such a way that pushing on the cap towards the skin of the subject, inserts the part that is to be inserted and the movement of the cap away from the skin of the subject initiates the retraction of the portion of the medical device that is to be retracted.

The retractor control element may comprise a locking portion which is in contact with an engagement portion of the cap, which prevents rotation of the retractor control element as long as the cap is positioned in its most distal position. When shipping the device or preparing the device for the insertion process, the cap typically will be in this most distal position. It is optionally provided to prevent at that moment an unintentional retraction movement of the retractor. Therefore, it is provided that the retractor control element may not rotate as long as the cap is in its most distal position. This also helps to improve the results of the insertion device in a drop test in which the device is dropped on a hard surface with the elastic element being pre-biased. Slight mechanical deformations in the drop test do not lead to an unintended retraction process prior to insertion. The contact between the locking portion of the retractor control element and of the engagement portion of the cap is lost once the cap is moved from its most distal position towards its most proximal position. The contact may be established, for example, for the movement of the cap towards its most distal position of five to ten percent of its movement such as a distance in a range between 0.5 to 3mm.

The retractor control element may be mounted rotatably on the inserter sleeve. By being mounted on the inserter sleeve, the retractor control element is well positioned with respect to the retractor, as the retractor may be provided within and be held and/or be guided by the inserter sleeve.

The movement of the cap with respect to the guide sleeve may be provided such that only a linear movement relative to the guide sleeve is possible by the cap. This allows for an easy to use insertion device as no complex movements of the cap with respect to the guide sleeve are necessary. In order to define this linear movement, (straight) guide portions may be provided on the cap and second guide portions co-operating with the first guide portions may be provided on the guide sleeve. Optionally, there are at least three such pairs of guide portions which allows to define a relative movement without blocking or wobbling of the two parts with respect to each other. For that purpose, it is optionally possible that the guide portions are equally spaced with respect to each other, such as at equally distanced circumferential positions.

The cap may be provided with pushing portions for pushing the inserter sleeve towards the proximal end of the insertion device and optionally also with separation limiting means which limit a separation of the cap away from the inserter sleeve towards the distal end of the insertion device. The pushing portions allow to translate the movement of the cap from its distal position towards the proximal position, also into a movement of the inserter sleeve from a distal position towards a proximal position. This movement of the inserter sleeve from a distal position to a proximal position allows to perform the insertion process.

The separation limiting means avoid that the cap may separate itself from the inserter sleeve in an uncontrolled fashion. The pushing portions together with the separation limiting means may define the allowed relative movement of the cap with respect to the inserter sleeve. This allowed relative movement may be between 1 to 5mm or between 1 and 3mm.

The cap, the inserter sleeve, the retractor and the retractor control element as well as the elastic element, may be together moveable from a distal position to a proximal position, at least along a portion of the movement of the cap; this movement takes places for at least a portion without a relative movement of the five parts with respect to each other. Along another portion of the movement of the cap at least the retractor control element may perform a purely rotational movement with respect to the other parts while the other parts move without a relative movement relative to each other.

The elastic element such as the spring may be provided in the pre-loaded state in which the spring is pressed together in order to store the energy for the retraction process. The elastic element may be provided to act between the retractor and the inserter sleeve. This means that the elastic element provides a force between the retractor and the inserter sleeve which may cause the retractor to move relative to the inserter sleeve once the movement path of the retractor is freed by the retractor control element.

The insertion system comprises an insertion device as described above or below and furthermore, it comprises the medical device to be inserted with the insertion device.

Possible embodiments of the insertion device are shown in the enclosed figures.
Figure 1a: an exploded view of an insertion device
Figure 1b: Details of some parts of the exploded view.
Figure 2a to Figure 2c: different views of the inserter sleeve, the retractor and the retractor control element.
Figure 3a, Figure 3b: different sectional views of the cap, the guide sleeve and the inserter sleeve.
Figure 4: a sectional view of an insertion device
Figure 5a, Figure 5b: different 3D views of the insertion device
Figure 6: sectional views of different states of the insertion device during the insertion process.

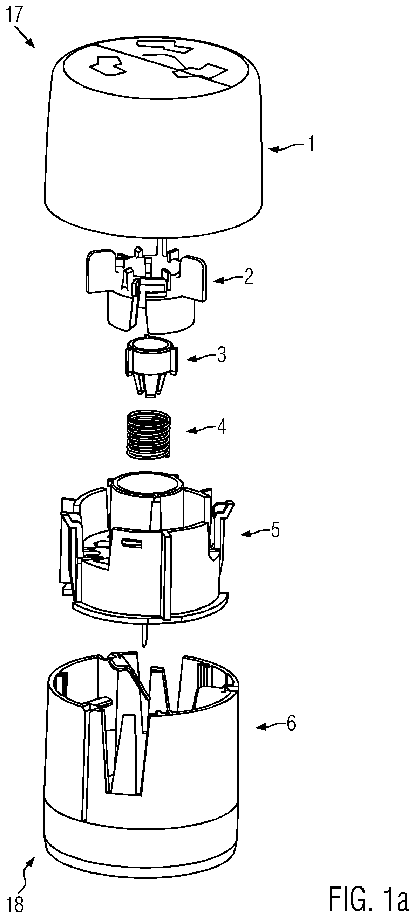

Figure 1a shows an exploded view of different parts of an embodiment of an insertion device. The insertion device may comprise a cap 1, a retractor control element 2, a retractor 3, an elastic element 4, an inserter sleeve 5 and a guide sleeve 6.

The cap 1 may be provided with an essentially cylindrical outer shape. The side wall of the cap may have a cylindrical or (slightly) conical shape. On the distal end 17 of the insertion device, the cap may have an essentially flat upper surface which serves as a surface on which a user can apply force by hand. The cap 1 may provide an inner volume into which other parts of the insertion device may be accommodated.

The retractor control element 2, the retractor 3, the elastic element 4 and the inserter sleeve 5 will be explained in more detail in the following figures. The guide sleeve 6 is provided with (essentially) cylindrical side wall and is open at its distal and proximal end. The cap 1, together with the guide sleeve 6, provide the housing of the insertion device. They are forming a joint inner space in which the retractor control element 2, the retractor 3, the spring 4 and the inserter sleeve 5 are provided.

The insertion device has a distal end 17 and a proximal end 18. The proximal end 18 is supposed to be placed in contact with the skin of a subject.

The insertion process may be performed by placing the proximal end 18 of the guide sleeve onto the skin of a subject and by exerting force e.g. by pressing on the distal top surface of the cap 1 onto the cap 1 and by moving the cap 1 towards the proximal end 18 or in other words towards the guide sleeve 6. The insertion process is terminated (as will be detailed further below) by the cap 1 being slightly moved in a distal direction (towards the distal end 17) after having been pressed on.

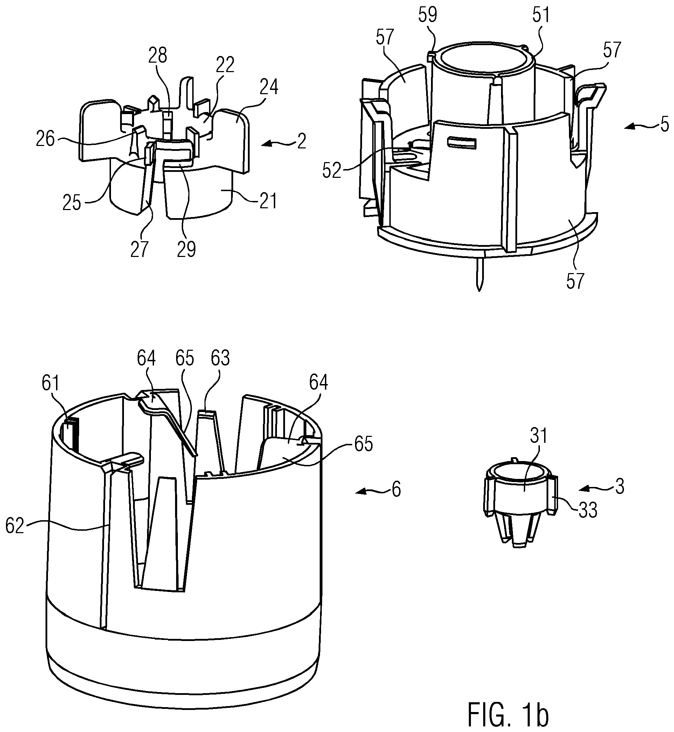

In Fig. 1b some parts shown in Fig. 1a are displayed separately and are provided with some reference numerals used further below. The reference numerals of Fig. 1b appears in other figures as well and Fig. 1b allows for a better understanding of the configuration of the distinct parts shown in the following figures.

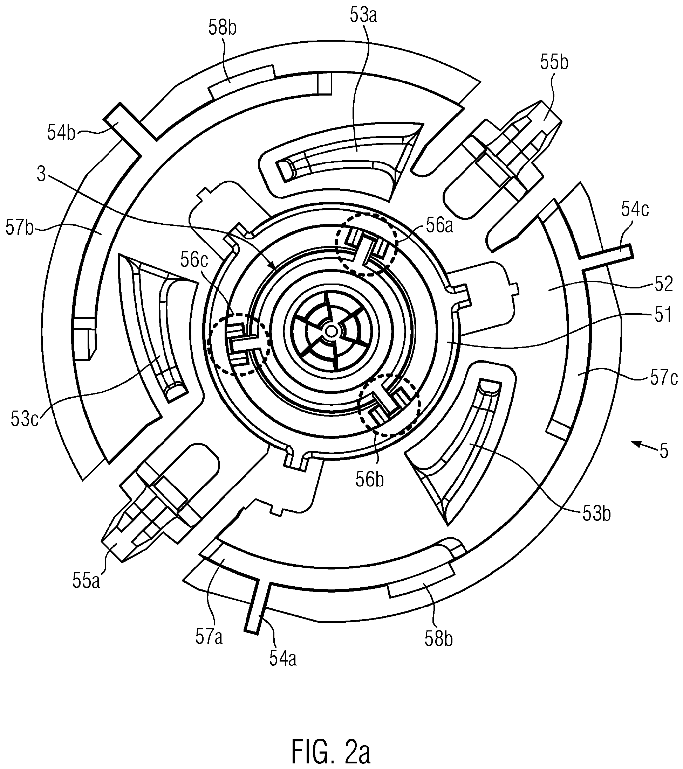

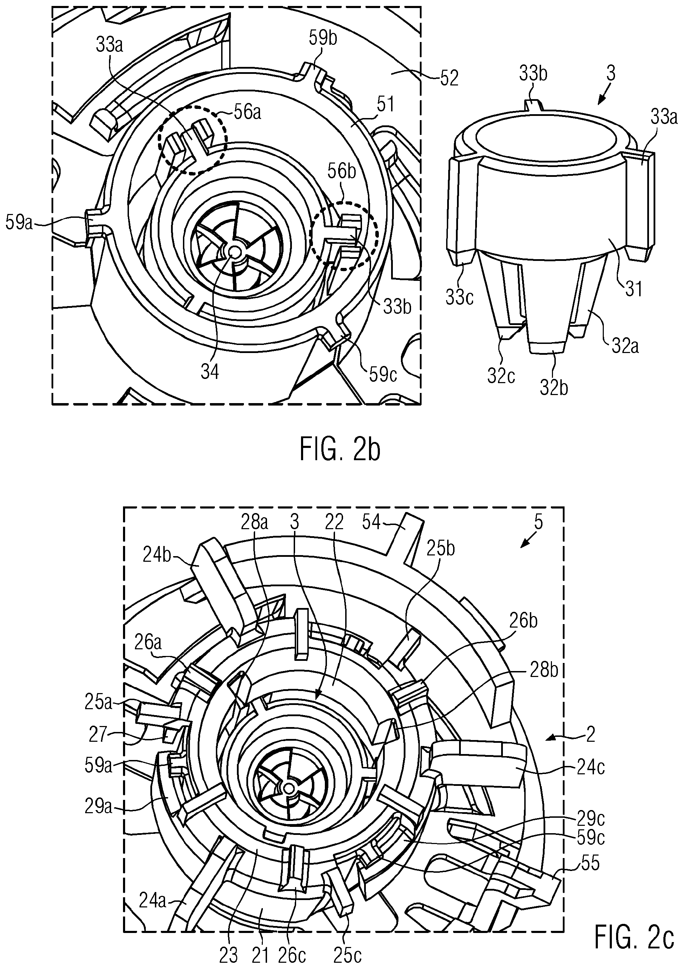

In figure 2a, details of the inserter sleeve 5 and the retractor 3 are shown. The view in figure 2a is provided onto the inserter sleeve 5 in the direction from the distal end 17 towards the proximal end 18. The inserter sleeve 5 may be provided with an inner sleeve portion 51 in which the retractor 3 is provided. The inserter sleeve 5 may provide one or more guide portions such as three or more guide portions 56a, 56b, 56c which in figure 2 are exemplary shown as a negative rail portion which provide a slot. The slot may also be provided without two negative rails but by a depression into a portion of the inserter sleeve 5 such as a depression in the inner sleeve portion 51. The guide portions may provide a part of the movement path of the retractor. Another part of the movement path of the retractor maybe provided by guide portions of the retractor control element.

The inserter sleeve 5 may, furthermore, be provided with an outer sleeve member 57 having several outer sleeve member parts such as parts 57a, 57b and 57c which can be called wall portions of the inserter sleeve 5. The outer sleeve member 57 is not an entirely closed sleeve but may comprise several openings or interruptions. The wall portions 57 may be provided on a cylindrical arrangement. The inserter sleeve 5 may furthermore have a disc shaped element 52, which may connect the different functional portions of the inserter sleeve. It may, for example, connect the inner sleeve portion 51 to the outer sleeve portion(s) 57. The disc-shaped element 52 may provide support for beams 53 which may be provided by the inserter sleeve 5. The inserter sleeve 5 may comprise one or more, two or more or three or more beams 53a, 53b, 53c which allow to elastically bias the cap 1 towards the distal end 17 as will be explained further below.

The inserter sleeve 5 may further comprise one or more such as two or more or three or more guide portions 54a, 54b, 54c which co-operate with guide portions of the guide sleeve 6 as will be explained further below. The guide portions 54a, 54b and 54c may be provided at equal distance with respect to each other (or at least two of the distances between the three guide portions 54a, 54b, 54c may be equal), as this provides for a safe way to avoid blocking of the movement of the inserter sleeve 5 with respect to the guide sleeve 6. They may, for example, be provided by an equal circumferential separation.

The disc-shaped element 52 may have an essentially circular outer perimeter.

The inserter sleeve 5 may, furthermore, provide device retention hooks 55a and 55b. There may also be more than two device retention hooks 55, while in figure 2a only two device retention hooks 55a and 55b are shown. The device retention hooks 55 serve to hold the medical device 7 prior and during the insertion process. The device retention hook(s) are an example of a holder for the medical device 7. It may release the medical device 7 after or at the end of the insertion process.

The inserter sleeve 5 may further comprise one or more, such as two or more or three or more openings (without reference signs) which are provided for the separation limiting rib 15 to reach through the inserter sleeve 5 and engage with the inserter sleeve 5 from the back side shown in figure 2a which is the proximal end 18 side.

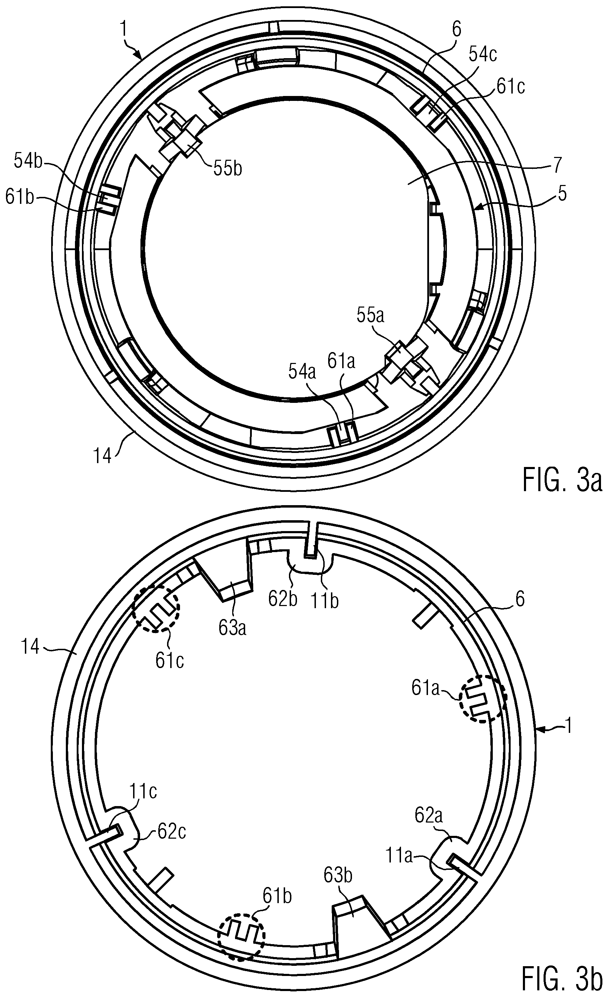

In figure 2b the retractor 3 is shown. The retractor 3 has a retractor body 31 which may be provided with an essentially cylindrical form. The retractor body 31 may be provided as a sleeve. On its outside, the retractor 3 may have guide portions 33 such as two or more or three or more guide portions 33a, 33b, 33c. The guide portions 33 may have an end (proximal end or lower end in Fig. 3) which is tapered which allows an easy assembly of the retractor 3 in the inserter sleeve 5. On its proximal end the retractor 3 may have hooks 32. In figure 2b the retractor 3 is provided with three hooks but there may be only two hooks or four or more hooks as well. The hooks 32a, 32b and 32c may be provided in order to engage with a portion 8 provided with the medical device 7 which is to be retracted. The retractor 3 may further be provided with a center portion 34 which prevents a movement of a portion 8 that is to be retracted towards the distal end 17. As further shown in figure 2b, the guide portions 33 may co-operate with the guide portions 56 of the inserter sleeve 5 in order to provide for a linear (only) movement of the retractor 3 with respect to the inserter sleeve 5.

As further shown in figure 2b, the inserter sleeve 5 may further be provided with coupling protrusions 59 such as two or more or three or more coupling protrusions 59a, 59b, 59c. These coupling protrusions 59 are provided in order to define a (rotational or purely rotational) movement of the retractor control element 2 with respect to the inserter sleeve 5. The coupling protrusions 59a, 59b, 59c may be provided on the outer side of the inner sleeve portion 51.

In figure 2c, a 3D view of the retractor control element 2 is shown in combination with the retractor and the inserter sleeve 5. The main body of the retractor control element 2 may be provided with an outer sleeve member 21 and an inner sleeve member 22 which may be connected by a connection member 23. The connection member 23 may be provided at the distal end of the outer sleeve member 21 and the inner sleeve member 22.

The retractor control element 2 may be provided on its outside with protruding portions 24, which may be used to provide a force to the retractor control element 2 which rotates the retractor control element 2. The protruding portion 24 may be provided at the outside of the retractor control element 2, which allows for a finer dosing and control of the force that creates the rotational movement but they may also be provided on its distal end or on its inner side. The retractor control element 2 has, essentially, a ring shaped body. This ring shaped body may comprise the outer sleeve member 21, the inner sleeve member 22 and the connection member 23.

In the embodiment shown in figure 2c, the retractor control element 2 has three protruding portions 24a, 24b and 24c. It may also have or also be possible to provide only two or four or more protruding portions 24. In the embodiment shown in figure 2c, the protruding portions 24a, 24b, 24c are provided with equal distances with respect to each other. This allows to ensure a smooth rotational movement without blocking of the retractor control element 2 during its rotational movement. It may also be that only two of the distances between different protruding portions 24 are equal.

The retractor control element 2, furthermore, may be provided with one, two, three or more or locking portions 25. These locking portions 25 are provided on the outside of the main body but they may also be provided on the distal end 23 or on the inner side of the main body. The locking portions 25 can prevent a rotation of the retractor control element 2 in combination with the cap, as will be explained further below.

The retractor control element 2 may be provided with one, two, three or more blocking protrusions 26. In figure 2c, three of such blocking protrusions 26, as shown. These protrusions 26a, 26b, 26c are shown on the distal end of the main body and in particular, on the connection member 23. They may also be provided on the outer side such as on the outer sleeve member 21 or on the inside such as on the inner sleeve member 22.

The retractor control element 2 may be provided to sit rotationally slidable on the inserter sleeve 5 and in particular on a dedicated portion of the inserter sleeve 5. In particular the retractor control element 2 may be provided to sit rotationally slidable on the inner sleeve portion 51 of the inserter sleeve 5. In this arrangement, the inner sleeve member 22 of the retractor control element 2 is provided on the inside of the inner sleeve portion 51 of the inserter sleeve 5, the connection member 23 is provided on the distal end of the inner sleeve portion 51 of the inserter sleeve 5 and the outer sleeve member 21 is provided outside of the inner sleeve portion 51 of the inserter sleeve 5. Thereby, the good support and guiding of the retractor control element 2 is provided for a rotational movement.

In order to avoid a separation of the retractor control element 2 from the inserter sleeve 5 by a movement of the retractor control element 2 towards the distal end 17 of the insertion device, the coupling protrusions 59 are provided on the inserter sleeve 5 which prevent a movement of the retractor control element 2 towards the distal end 17. In figure 2b and 2c these coupling protrusions 59a, 59b and 59c are provided on the outside of the inner sleeve 51 but they may also be provided on the inside, thereof.

Co-operating with the coupling protrusions 59, there may be provided slots 29 in the retractor control element 2 which accommodate the coupling protrusions 59. The bottom of the slots 29 thereby are in contact with the bottoms of the coupling protrusions 59. The retractor control element 2 may, furthermore, be provided with an assembly slot 27 which connects the slots 29 with the proximal end of the retractor control element 2. Thereby, the protrusions 59 of the inserter sleeve 5 may be brought into the slots 29 during assembly of the insertion device.

The bottom surface(s) of the retractor control element 2 may be provided to come in contact with guide portions of the retractor 3. This may refer in particular to the guide portions 33 as explained above. The bottom surface(s) of the retractor control element 2 is/are a surface/surfaces facing towards the proximal end 18 of the insertion device 1..6. As long as the guide portions 33 of the retractor 3 come in contact with the bottom surface(s) of the retractor control element 2, the retractor 3 is blocked from moving in the direction towards the distal end 17.

The retractor control element 2, furthermore, has guide portions 28 which do allow a movement of retractor 3 towards the distal end 17 as soon as these guide portions 28 are aligned with the guide portions 33 of the retractor 3. As shown in figure 2c the retractor control element 2 is in a position where the guide portions 28 are not in alignment with the guide portion 33 and the retractor 3 is blocked from moving towards the distal end 17.

The guide portions 28 (28a, 28b, 28c) may be provided as one, two, three or more guide portions. If more than one guide portion 28 is provided then those guide portions or some of the guide portions 28 are preferably separated by an equal distance with respect to each other. This avoids an unintentional blocking of the retractor 3 while moving relative to the contractor control element 2.

The retractor control element 2 may be arranged such that it can be rotated into a position in which the guide portions 28 are aligned with the guide portions 56 of the inserter sleeve. Thereby, the movement of the retractor towards the distal end 17 can be enabled. This is done by freeing the moving path of retractor 3 in the direction of the distal end 17. The movement path is in this case provided by the combination of the aligned guide portions 28 of the retractor control element and the guide portions 56 of the inserter sleeve.

In figure 3a a view onto the proximal end 18 of the insertion device in the direction towards the distal end 17 is shown. The most outer part is the proximal end of the cap 1. Inside of the cap 1 is shown the guide sleeve 6 with its proximal end surface. Guide portions 61 of the guide sleeve are shown which co-operate with guide portions 54 of the inserter sleeve. In the embodiment shown in figure 3a, there are three pairs of guide portions 54, 61 namely guide portions 54a, 54b and 54c co-operating with guide portions 61a, 61b, 61c, respectively. The guide portions are arranged at equal distances with each other. As can be seen, the insertion device may have an essentially circular cross section.

In figure 3a, the bottom surface of the medical device 7 is shown. This surface is supposed to come in contact with the skin 9 of the subject. On this bottom surface of the medical device 7 e.g. an adhesive may be provided which sticks the medical device 7 to the skin 9 of the subject.

In the middle of the medical device 7, a needle and/or a sensor may be provided that need to be inserted through the skin 9 of the subject. In figure 3a, it is shown that the inserter sleeve 5 may be provided with device retention hooks 55. In the embodiment in figure 3a, two of such device retention hooks 55, namely 55a, 55b are shown. However, it is also possible to provide only one or three or more device retention hooks 55. During the insertion process, the device retention hooks 55 move outwardly, thereby freeing the medical device 7 from its position within the insertion device. The two device retention hooks may be provided opposite to each other with respect to the center of the insertion device.

Figure 3b shows a view from the relation of the cap 1 with respect to the guide sleeve 6. The cap 1 is provided on its inside, in particular on the inside of the housing portion 14, with guide portions 11. As shown in the embodiment in figure 3b, there are provided three such guide portions 11, namely 11a, 11b and 11c. The guide portions 11 can co-operate with corresponding guide portions 62 provided by the guide sleeves 6. In the embodiment shown in figure 3b, the guide portion 11 is provided as a positive rail and the guide portion 62 of the guide sleeve 6 as a negative rail or as a slot. The inverse arrangement is possible as well in which the guide portions 62 of the guide sleeve 6 are a positive rail and the guide portions 11of the cap ar a negative rail/slot.

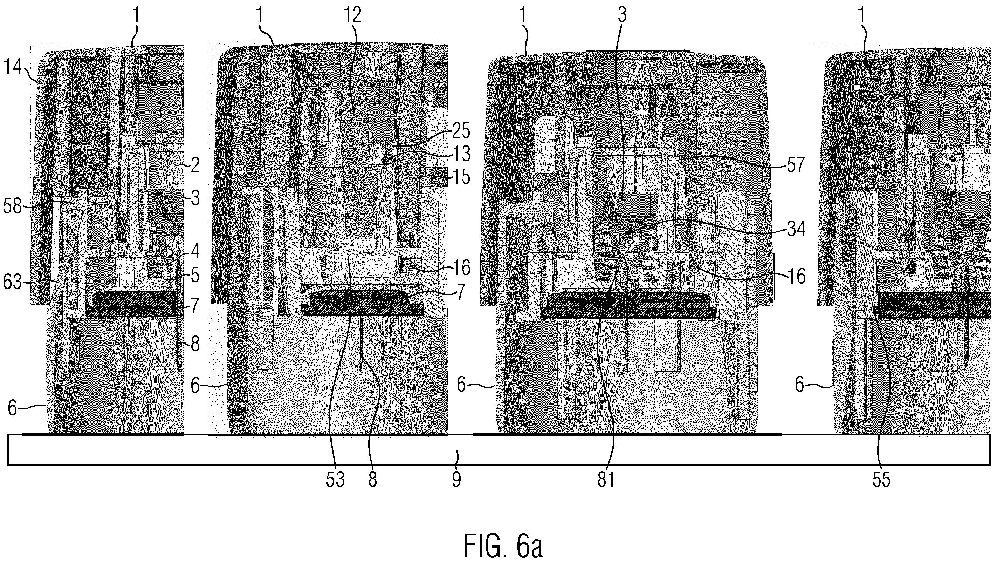

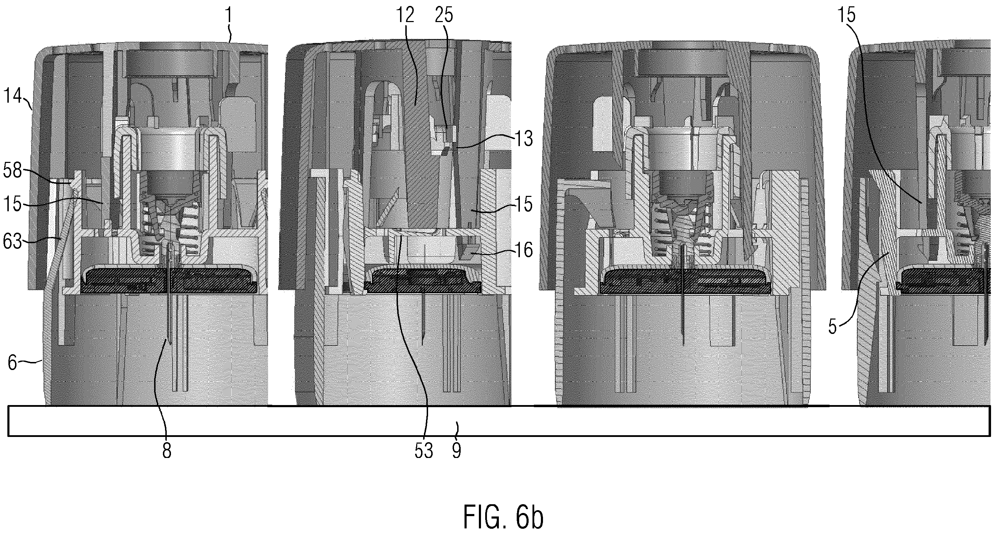

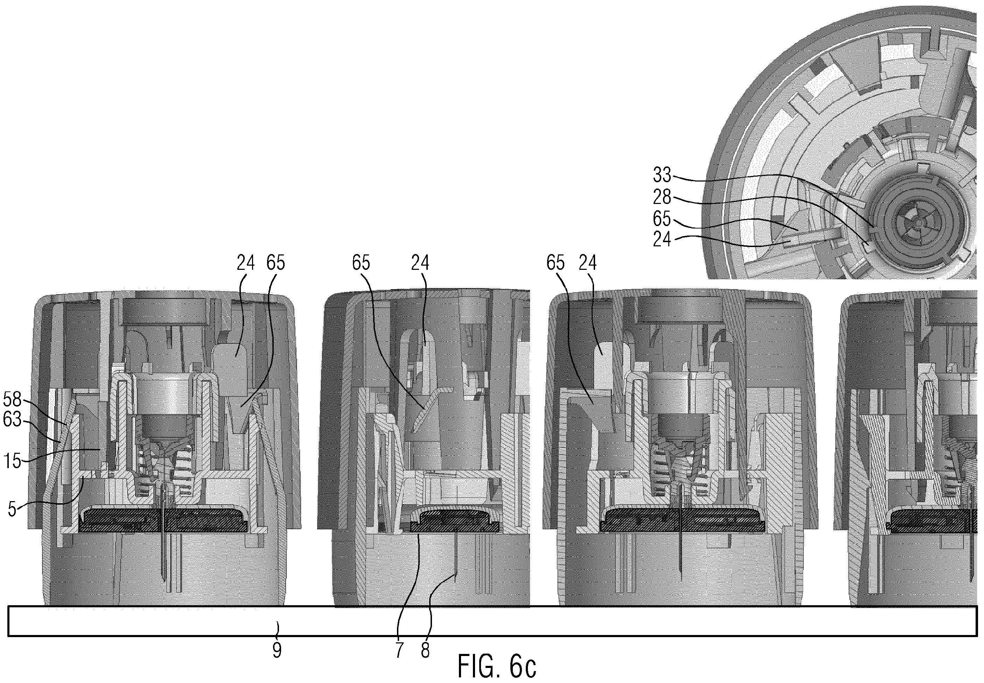

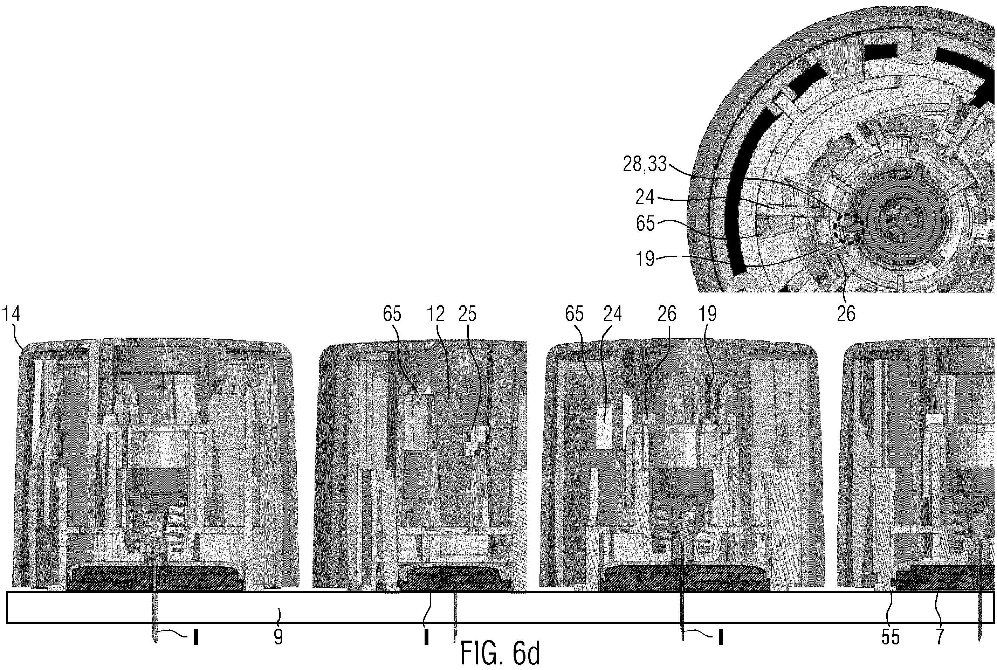

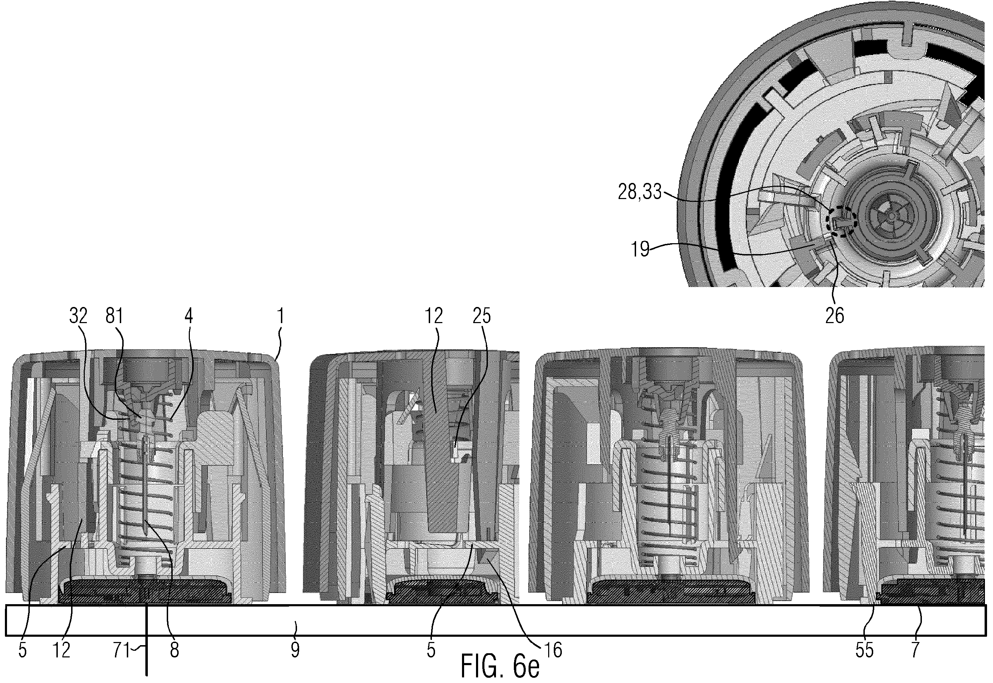

Furthermore, shown in figure 3b are guide portions 61 on the inside of the guide sleeve. Those guide portions are already discussed in relation with figure 3a. In figure 3b, it is shown that the guide sleeve 6 may comprise one or more retainer beams 63. Such retainer beams 63 maintain the inserter sleeve 5 in a distal position unless a certain amount of force is applied to the cap 1 towards the proximal end 18. This will be explained in further detail in relation to figure 6.

As can be seen in figure 3b, the guide sleeve 6 can have an essential circular cross section.

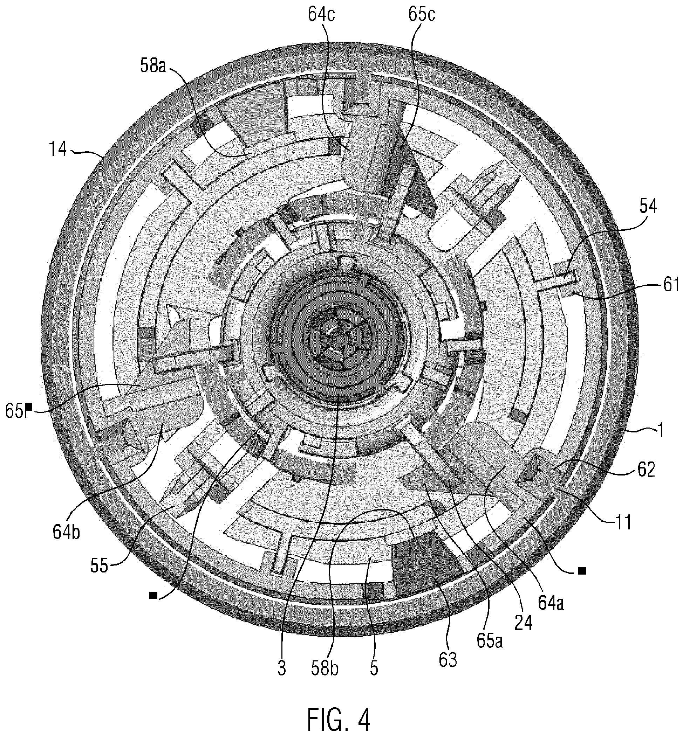

Figure 4 shows a view from the distal end 17 towards the proximal end 18 of the insertion device with the cut just above the retractor control element 2. In this view, ramps 65 are shown which can co-operate with protruding portions 24 as will be discussed further below.

In the view in figure 4, it is furthermore shown that inner portions of the cap 1 co-operate with parts of the retractor control element 2 which limit, or allow, or block, or free a rotational movement of the retractor control element as will be discussed further in relation to figure 6.

As can be seen in figure 4, the ramps 65 can be provided as an inner part of the guide sleeve 6 and each ramp can be connected to remaining parts of the guide sleeve 6 by a ramp support 64.

As shown in figure 4, there can be three ramps 65, namely 65a, 65b, 65c. It is also possible to have fewer than three, such as one or two ramps, or more than three ramps. As the ramps 65 co-operate with the protruding portions 24 of the retractor control element 2 the number of such protruding portions 24 of the retractor control element 2 and the number of ramps 65 of the guide sleeve 6 may be provided in the same number. In the embodiments shown in figure 4, there are three ramps 65 co-operating with three protruding portions 24.

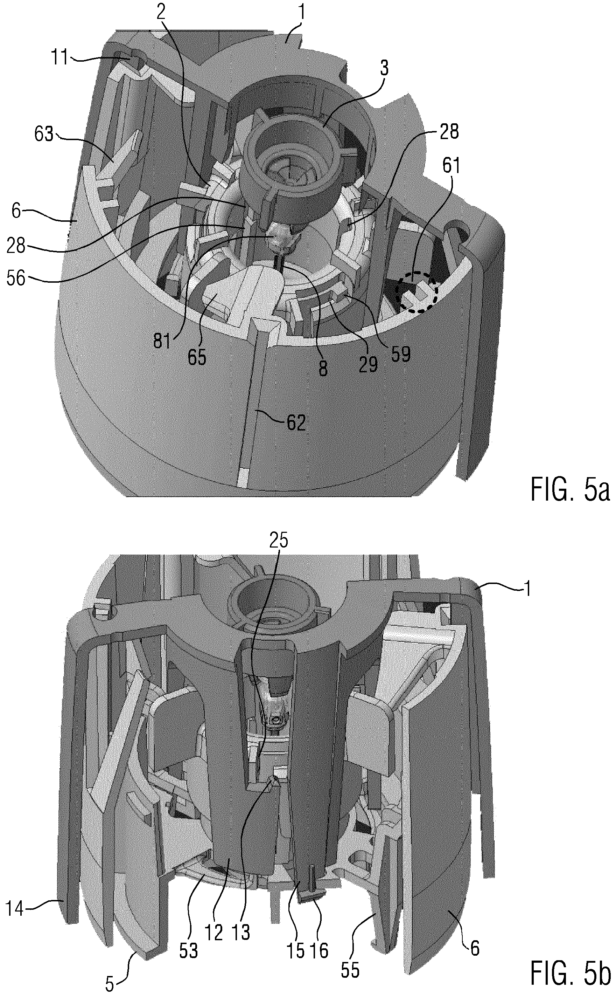

Figure 5a shows a three-dimensional view into the insertion device with parts of it removed in order to make interior components visible.

As can be seen in figure 5a on the outside of the essentially circumferential wall portion of the guide sleeve 6 negative rails or slots 62 are provided which guide the guide portions 11 of the cap 1. Furthermore, the guide portions 61 on the inside of the guide sleeve 6 are shown. Furthermore, in figure 5a it is shown that ramps 65 may be provided as part of the guide sleeve 6 and facing towards the inner part of the insertion device.

In figure 5a, the retractor 3 is shown in a position in which it has moved towards the distal end 17 of the insertion device, as the retractor 3 is shown above the retractor control element 2. In figure 5a the spring 4 which has pushed the retractor 3 towards the distal end 17 is not shown for clarity.

In figure 5a, it is shown that the retractor 3 can be in engagement with the head of a portion that is to be retracted - here head 81 of the needle 8.

In the configuration shown in figure 5a, the guide portions 28 of the retractor control element 2 are shown in alignment with the guide portions 56 of the inserter sleeve 5 that define (at least a portion of) the movement path of the retractor 3. In that position, the protrusion 59 is at the end of the slot 29 of the retractor control element 2, thereby limiting the rotational movement of the retractor control element beyond the alignment of the guide portions 28 with those guide portions 56 of the inserter sleeve 5.

Figure 5b provides another view into the insertion device with other parts removed in order to provide visibility of interior portions. In figure 5b in particular, it is shown that the cap 1 may be provided with a pushing portion 12 which extends from the top surface of the cap 1 at its distal end towards the proximal end of the insertion device. This pushing portion 12 is arranged such that it can come into contact with beams 53 of the inserter sleeve 5. Furthermore, the pushing portion 12 can also come into contact with other parts of the inserter sleeve 5, allowing to push the inserter sleeve 5 towards the proximal end 18 of the device during the insertion process. As shown in figure 5b, the cap 1 may further be provided with an engagement portion 13 which interacts with the locking portion 25 provided on the retractor control element 2. The engagement portion 13 may prevent a rotation of the retractor control element 2. This may be the case, at least as long as the cap 1 is in its most distal position.

In figure 5b it is furthermore shown that the cap 1 may comprise a separation limiting rib 15 which at its lower end may be provided with a separation limiting hook 16. This separation limiting rib 15 can extend from the top surface of the cap 1 at its distal end towards the proximal end 18 of the insertion device. It thereby reaches through an opening in the inserter sleeve 5, such that the separation limiting hook 16 can come into contact with the surface of the inserter sleeve 5 facing towards the proximal end 18 of the insertion device. In this way, the uncontrolled separation of the cap 1 into the distal direction 17 away from the inserter sleeve 5 is prevented.

The pushing portion 12 in co-operation with the separation limiting rib 15 and the separation limiting hook 16 may provide or define a limited movement range of the cap 1 with respect to the inserter sleeve 5, the movement of those two portions relative to each other may be limited to a range between 0.5 and 2mm, for example.

In the configuration shown in figure 5b, the locking portion 25 is not in contact with the engagement portion 13 but, as the retractor control element 2 has already been rotated, the locking portion 25 now comes into contact with a portion of the cap 1 such as the pushing portion 12 or the separation limiting rib 15. Thereby, the rotation of the retractor control element 2 beyond the position in which the guide portions of the retractor control element and of the inserter sleeve 5 are aligned is prevented.

In figure 6a to 6e, different sections through the insertion device are shown, wherein in each figure 6a to 6e, the different shown sections correspond to the same state in which the insertion device is shown.

Figure 6a shows a state of the insertion device in which the insertion device is provided with the medical device 7 to be inserted. The medical device 7 is shown at the proximal end of the inserter sleeve 5 and it is retained by device retention hooks 55 (shown in figure 6a, most right view). The medical device 7 is provided together with a needle 8. The needle 8 is to be inserted through the skin 9 of a subject. Together with the needle 8 may be provided an analyte sensor which is to be inserted through skin 9 of a subject.

The guide sleeve 6 is provided with its proximal end portion on the skin 9 of the subject.

In the configuration shown in figure 6a the inserter sleeve 5 is shown to be able to rest with its blocking portion 58 on retainer beam 63 of the guide sleeve. The spring 4 (elastic element) is in its preloaded state and compressed against its spring force. The spring 4 may rest with its proximal end in a ring shaped depression of the inserter sleeve 5. The distal end of the elastic element 4 is in contact with the retractor 3 and biases the retractor 3 in the distal direction (upward in figures 6).

In the orientation shown in figure 6, the lower end of the insertion device corresponds to its proximal end 18 which is the end that is in contact with the skin 9 and the distal end 17 refers to the upper end shown in figure 6a.

As can be seen in figure 6a, the retractor 3 is in an engaged relation with the head 81 of a needle 8. This engagement relation can be achieved by inserting the medical device together with the head 81 into the inserter sleeve 5. The medical device 7 thereby opens the retention hooks 55 and as they snap back into a closed position they retain the medical device 7 in the position shown in figure 6a. When adding the medical device 7 to the insertion device the head 81 of the needle 8 also opens the hooks 32, and as the head 81 has come into contact with the center portion 34 of the retractor, the hooks 32 snap back inwardly and thereby retain the head 81.

As shown in figure 6a in the second section from the left, cap 1 is pushed upwards by the pushing portion 12 which rests on beam 53. This beam 53 is slightly elastic and elastically pushes the cap 1 into its most distal (upward in figure 6a) position.

The separation limiting hook 16 may be in contact with a lower surface of the inserter sleeve 5 and thereby prevents a further upward movement of the cap 1 in relation to the inserter sleeve 5.

As furthermore shown in the second intersection from the left, the locking portion 25 is in contact with the engagement portion 13 and thereby prevents rotation of the retractor control element 2. The position shown in figure 6a may be the starting position with respect to the rotational movement of the retractor control element 2. When viewed from the top i.e. in the direction from the distal end 17 towards the proximal end 18, the rotation of the retractor control element 2 is in the clockwise direction as will be discussed further below.

The right most intersection shows that the inserter sleeve 5 may be provided with a device retention hook 55 which retains the medical device 7 in its position as shown in figure 6a.

As long as the cap 1 is in its upmost position (most distal position) shown in figure 6a, the contact of the locking portion 25 with the engagement portion 13, prevents the rotation of the retractor control element 2 also in case the device as shown in figure 6a falls down and drops on a surface such as a table or a floor. The engagement portion 13 may allow the rotation of the retractor control element 2 only once the cap 1 is moved into a lower position as will be discussed in the following.

In figure 6b the situation is shown in which some force or some pressure has been applied to the top surface of the cap 1. The inserter sleeve 5 still remains in its original position as the blocking portion 58 still rests on the retainer beam 63. However, as can be seen in this figure 6b, the cap 1 may now be in contact with the inserter sleeve 5 such that the force applied to the cap 1 translates into an equal force onto the inserter sleeve 5. In the situation shown in figure 6b it can be seen that the pushing portion 12 can have slightly bent the beams 53 against the small force (smaller than the force provided to press on the cap 1) provided by the beams 53.

Further, it can be seen that the separation limiting hooks 16 has lost contact with the lower surface of the inserter sleeve 5 as the cap 1 has travelled down a little bit with respect to the inserter sleeve 5. This can be seen in particular in the three most right sections shown in figure 6b.

As can be seen in figure 6b as the cap 1 has moved downward relative to the retractor control element 2 the locking portion 25 is no more in contact with the engagement portion 13 of the cap and thereby the rotational movement of the retractor control element 2 is made possible.

After having applied further pressure on the cap 1, the blocking portions 58 do no more rest on the retainer beams 63 but have passed below them (see figure 6c). The force on the cap 1 may have translated to a force onto the inserter sleeve 5 by e.g. a contact of a pushing portion 12 of the cap 1 with the inserter sleeve 5. As can be seen in particular in the second section from the left the protruding portion 24 now comes into contact with the ramp 65 of the inserter sleeve 5. As the surface of the ramp 65 is inclined (With respect to a direction perpendicular to skin onto which the insertions device can be placed and/or with respect to a longitudinal axis of the insertion device and/or with respect to the direction of the linear movement of the cap towards proximal end 18), the downward movement of the retractor control element 2 translates into rotational movement of the retractor control element 2. As can be seen in the upper inset in figure 6c, the guide portions 28 of the retractor control element 2 are not yet in alignment with the guide portions 33 of the retractor 3.

As the inserter sleeve 5, the elastic element (spring) 4, the retractor 3 and the retractor control element 2 together with the cap 1 travel further down and make a relative movement with respect to the guide sleeve 6, the protruding portions 24 rotate further in sliding contact with the ramp 65 and the rotation of the retractor control element 2 progresses as the cap 1 of the retractor control element 2, the elastic element (spring) 4 and the inserter sleeve 5 travel further down in figure 6.

In figure 6d, a state is shown in which the cap 1 has reached its lowest most position. The cap 1 now can rest on the guide sleeve 6. The needle 8 has penetrated the skin 9 of the subject and the bottom surface of the medical device 7 has come into contact with the skin 9 of the subject. In case that this bottom surface is provided with an adhesive the portion of the medical device 7 may adhere to the skin 9 of the subject. In that position the guide sleeve interacts with a portion of the inserter sleeve 5 which opens the device retention hook 55 (this is not shown in figure 6d).

As can be seen in the third intersection from the left, the protruding portions 24 have passed the lower end of the ramps 65 and the ramps (such as the lower end of the ramps) provide a slightly elastic force onto the protruding portions 24 into the rotational direction of the retractor control element 2.

In the situation in figure 6d it is shown that blocking protrusion 26 of the retractor control element 2 may come in contact with a blocking portion 19 of the cap 1 which prevents the rotational movement of the retractor control element 2 to complete. This can also be seen in the upper inset in figures 6d in which it can be seen that the guide portions 28 are not yet in full alignment with the guide portions 33 of the retractor 3.

In figure 6e the situation is shown in which the cap 1 is slightly moved upwards (in the distal direction). In this configuration the blocking portion 19 may lose contact with the blocking protrusion 26 so that the retractor control element 2 may complete its rotational movement into a position as shown in the upper inset as shown in figures 6e. In this position as shown the guide portions 28 may come in full alignment with the guide portions 33 of the retractor 3 such that by the force of the elastic element 4 the retractor now travels in the distal direction (upwardly in Fig. 6d). This movement may move the retractor 3 through the retractor control element 2.

The movement of the retractor 3 caused by the elastic element 4 may push the retractor 3 through the retractor control element 2 to an extent that the retractor 3 loses contact with the retractor control element 2. The retractor thereby may leave the movement path that may be considered to be defined at least in part by the guide portions of the retractor control element 2.

The retractor control element 2 completes its rotation due to the force exerted by the lower end portions of the ramps 65 onto the protruding portions 24.

The upward movement of the cap 1 in figure 6e is limited by the separation limiting hook 16 coming into an engagement with the inserter sleeve 5.

The needle 8 is retracted into a position such that a contact of the needle 8 with a user can be avoided by being retracted within the insertion device.

After the needle 8 being retracted, a sensor 71 of the medical device 1 may remain inserted through the skin 9 of the subject. The needle 8 is no longer in contact with the medical device.

The insertion device 1..6 may then be lifted from the medical device 7 which may stay in contact with the skin 9 of the subject and the insertion process is completed.

### List of reference signs:

**1 Cap**
   11 guide portions, rail
   12 pushing portion
   13 engagement portion
   14 housing portion
   15 separation limiting rib
   16 separation limiting hook
   17 distal end
   18 proximal end
   19 blocking portion
**2 Retractor control element**
   21 outer sleeve member
   22 inner sleeve member
   23 connection member
   24 protruding portion
   25 locking portion
   26 blocking protrusion
   27 assembly slot
   28 guide portion, negative rail
   29 slot
**3 Retractor**
   31 retractor body
   32 hooks
   33 guide portions, positive rail
   34 centre portion
**4 Elastic element**
**5 Inserter sleeve**
   51 inner sleeve portion
   52 disk-shaped element
   53 beam
   54 guide portion
   55 device retention hook
   56 guide portion, negative rail
   57 outer sleeve member
   58 blocking portion
   59 coupling protrusion
**6 Guide sleeve**
   61 guide portion, negative rail
   62 guide portion, negative rail
   63 retainer beam
   64 ramp support
   65 ramp
**7 Medial device**
   71 sensor
**8 Needle**
   81 head of needle
**9 Skin**

## Claims

1. Insertion device (1..6) for at least partially inserting a medical device (7) including an analyte sensor through the skin (9) of a subject, the insertion device (1..6) having a proximal end (18) to be put in contact with the skin (9) and a distal end (17) opposite to the proximal end (18), the insertion device (1..6) comprising:
a guide sleeve (6) being provided at the proximal end (18) and to be placed in contact with the skin (9) for an insertion process;
a cap (1) being provided at distal end (17), which can be pressed on to carry out at least part of the insertion process while moving in the direction of the proximal end (18);
an inserter sleeve (5) provided at least partially within the guide sleeve (6) and/or the cap (1) and being provided with a holder (55) for the medical device (7) and the inserter sleeve (5) being moveable with respect to the guide sleeve (6);
a retractor (3) configured to retract a portion (8) provided with the medical device (7), the retractor (3) being moveable along a movement path (28, 56);
an elastic element (4) such as a spring, which can bias the retractor (3) towards the distal end (17) along the movement path (28, 56); and
a retractor control element (2) which controls the movement of the retractor (3) by blocking or freeing the movement of the retractor (3) along the movement path (28, 56) and which is itself moveable such as rotatable with respect to the retractor (3);
wherein the inserter device is **characterised in that** the retractor control element (2) is mounted rotationally slidable on an inner sleeve portion of the inserter sleeve (5).

2. Insertion device of claim 1, wherein retractor control element (2) is rotatable between a position in which it blocks the movement of the retractor (3) along the movement path (28, 56) and an other position in which it frees the movement of the retractor (3) along the movement path (28, 56).

3. Insertion device of claim 1 or 2, wherein the retractor control element (2) is rotatable by a portion (65) of the guide sleeve (6), which comes into contact with the retractor control element (2), such as with a protruding portion (24) of the retractor control element (2), upon a relative movement of the retractor control element (2) with respect to the guide sleeve (6) such as a movement of the retractor control element in the direction of the proximal end.

4. Insertion device of any of claims 1 to 3, wherein the movement of the retractor (3) along the movement path (28, 56) is confined to a linear movement in the direction of the proximal end (18) to distal end (17) or vice versa, the confinement to the linear movement optionally being provided by at least a pair of guide portions (33, 56), the pair of guide portions comprising a first guide portion such as a rail (33) and a cooperating second guide portion such as a negative rail (56), with one of the two guide portions (33, 56) being provided at the inserter sleeve (5) and the other guide portion (33, 56) provided at the retractor (3), wherein optionally two or three such pairs of guide portions are provided.

5. Insertion device of any of claims 1 to 4, wherein the retractor control element (2) is provided with at least one guide portion (28) which can be brought into alignment with another guide portion (56) such as the guide portion of claim 4 of the inserter sleeve (5) in order to free the movement of the retractor (3) along a portion of the movement path and blocking the movement of the retractor (3) along the portion of the movement path (28, 56) in case the guide portion (28) of the retractor control element (2) is not in such alignment.

6. Insertion device of any of claims 1 to 5, wherein the retractor control element (2) is provided with at least one blocking protrusion (26) which is provided to interact with a blocking portion (19) of the cap (1), the contact of the blocking protrusion (26) and the blocking portion (19) preventing rotation of the retractor control element (2) into a position in which it frees the movement path (28) of the retractor (3).

7. Insertion device of claim 6, wherein the contact of the at least one blocking protrusion (26) of the retractor control element (2) and the blocking portion (19) of the cap (1) is possible in a position of the cap (1) in its most proximal position upon being pressed on.

8. Insertion device of any of claims 1 to 7, wherein the retractor control element (2) comprises a locking portion (25) which is in contact with an engagement portion (13) of the cap (1) which prevents rotation of the retractor control element (2) as long as the cap (1) is positioned at its most distal position.

9. Insertion device of any of claims 1 to 8, wherein the cap (1) and the guide sleeve (6) are provided relatively to each other such that the cap (1) performs a linear movement relative to the guide sleeve (6), where the linear movement is guided by at least one pair of guide portions (11, 62), each pair of guide portions having a first guide portion (11) in the cap (1) and a cooperating second guide portion (62) on the guide sleeve (6), wherein the inserter device optionally is provided with at least three such pairs (11a, 62a, 11b, 62b, 11c, 62c) of guide portions, wherein the guide portions further optionally are provided equally spaced to each other.

10. Insertion device of any of claims 1 to 9, wherein the cap (1) is provided with one or more pushing portions (12) for pushing the inserter sleeve (5) towards the proximal end (18) and optionally with separation limiting means (15, 16) for limiting a separation of the cap (1) away from the inserter sleeve (5) towards the distal end (17).

11. Insertion device of any of claims 1 to 10, wherein the cap (1), the inserter sleeve (5), the retractor (3), the retractor control element (2) and the elastic element (4) are movable from a distal position to a proximal position together at least along a portion of the movement of the cap (1), optionally without a relative movement relative to each other.

12. Insertion device of any of claims 1 to 11, wherein the elastic element (4) is provided in a preloaded state acting between the retractor (3) and the inserter sleeve (5).

13. Insertion system comprising an insertion device (1...6) as of any of claims 1 to 12 and a medical device (7) to be inserted with the insertion device (1...6).

## Patentansprüche

1. Einführvorrichtung (1..6) zum mindestens teilweisen Einführen einer medizinischen Vorrichtung (7), die einen Analytsensor einschließt, durch die Haut (9) eines Subjekts, wobei die Einführvorrichtung (1..6) ein mit der Haut (9) in Kontakt zu bringendes proximales Ende (18) und ein dem proximalen Ende (18) gegenüberliegendes distales Ende (17) aufweist, wobei die Einführvorrichtung (1..6) Folgendes umfasst:
eine Führungshülse (6), die an dem proximalen Ende (18) vorgesehen ist und für einen Einführvorgang mit der Haut (9) in Kontakt zu bringen ist;
eine Kappe (1), die an dem distalen Ende (17) vorgesehen ist, auf die gedrückt werden kann, um mindestens einen Teil des Einführvorgangs auszuführen, während sie sich in Richtung des proximalen Endes (18) bewegt;
eine Einführhülse (5), die mindestens teilweise innerhalb der Führungshülse (6) und/oder der Kappe (1) vorgesehen ist und mit einem Halter (55) für die medizinische Vorrichtung (7) versehen ist, wobei die Einführhülse (5) in Bezug auf die Führungshülse (6) beweglich ist;
einen Retraktor (3), der konfiguriert ist, um einen mit der medizinischen Vorrichtung (7) versehenen Abschnitt (8) zurückzuziehen, wobei der Retraktor (3) entlang eines Bewegungswegs (28, 56) beweglich ist;
ein elastisches Element (4), wie z. B. eine Feder, das den Retraktor (3) entlang des Bewegungswegs (28, 56) zu dem distalen Ende (17) hin lenken kann; und
ein Retraktorsteuerelement (2), das die Bewegung des Retraktors (3) steuert, indem es die Bewegung des Retraktors (3) entlang des Bewegungswegs (28, 56) blockiert oder freigibt, und das selbst beweglich ist, wie z. B. in Bezug auf den Retraktor (3) drehbar ist;
wobei die Einführvorrichtung **dadurch gekennzeichnet ist, dass** das Retraktorsteuerelement (2) drehbar verschiebbar an einem inneren Hülsenabschnitt der Einführhülse (5) montiert ist.

2. Einführvorrichtung nach Anspruch 1, wobei das Retraktorsteuerelement (2) zwischen einer Position, in der es die Bewegung des Retraktors (3) entlang des Bewegungswegs (28, 56) blockiert, und einer anderen Position, in der es die Bewegung des Retraktors (3) entlang des Bewegungswegs (28, 56) freigibt, drehbar ist.

3. Einführvorrichtung nach Anspruch 1 oder 2, wobei das Retraktorsteuerelement (2) durch einen Abschnitt (65) der Führungshülse (6), der mit dem Retraktorsteuerelement (2) in Kontakt kommt, wie z. B. mit einem vorstehenden Abschnitt (24) des Retraktorsteuerelements (2), bei einer relativen Bewegung des Retraktorsteuerelements (2) in Bezug auf die Führungshülse (6), wie z. B. einer Bewegung des Retraktorsteuerelements in Richtung des proximalen Endes, drehbar ist.

4. Einführvorrichtung nach einem der Ansprüche 1 bis 3, wobei die Bewegung des Retraktors (3) entlang des Bewegungswegs (28, 56) auf eine lineare Bewegung in Richtung des proximalen Endes (18) zu dem distalen Ende (17) oder umgekehrt beschränkt ist, wobei die Beschränkung auf die lineare Bewegung optional durch mindestens ein Paar von Führungsabschnitten (33, 56) bereitgestellt wird, wobei das Paar von Führungsabschnitten einen ersten Führungsabschnitt, wie z. B. eine Schiene (33), und einen zusammenwirkenden zweiten Führungsabschnitt, wie z. B. eine negative Schiene (56), umfasst, wobei einer der zwei Führungsabschnitte (33, 56) an der Einführhülse (5) vorgesehen ist und der andere Führungsabschnitt (33, 56) an dem Retraktor (3) vorgesehen ist, wobei optional zwei oder drei solcher Paare von Führungsabschnitten vorgesehen sind.

5. Einführvorrichtung nach einem der Ansprüche 1 bis 4, wobei das Retraktorsteuerelement (2) mit mindestens einem Führungsabschnitt (28) versehen ist, der mit einem anderen Führungsabschnitt (56), wie z. B. dem Führungsabschnitt nach Anspruch 4 der Einführhülse (5), in Ausrichtung gebracht werden kann, um die Bewegung des Retraktors (3) entlang eines Abschnitts des Bewegungswegs freizugeben und die Bewegung des Retraktors (3) entlang des Abschnitts des Bewegungswegs (28, 56) zu blockieren, falls der Führungsabschnitt (28) des Retraktorsteuerelements (2) nicht in einer solchen Ausrichtung ist.

6. Einführvorrichtung nach einem der Ansprüche 1 bis 5, wobei das Retraktorsteuerelement (2) mit mindestens einem Blockiervorsprung (26) versehen ist, der vorgesehen ist, um mit einem Blockierabschnitt (19) der Kappe (1) zu interagieren, wobei der Kontakt des Blockiervorsprungs (26) und des Blockierabschnitts (19) eine Drehung des Retraktorsteuerelements (2) in eine Position verhindert, in der es den Bewegungsweg (28) des Retraktors (3) freigibt.

7. Einführvorrichtung nach Anspruch 6, wobei der Kontakt des mindestens einen Blockiervorsprungs (26) des Retraktorsteuerelements (2) und des Blockierabschnitts (19) der Kappe (1) in einer Position der Kappe (1) in ihrer proximalsten Position möglich ist, wenn diese gedrückt wird.

8. Einführvorrichtung nach einem der Ansprüche 1 bis 7, wobei das Retraktorsteuerelement (2) einen Verriegelungsabschnitt (25) umfasst, der mit einem Eingriffsabschnitt (13) der Kappe (1) in Kontakt steht, der eine Drehung des Retraktorsteuerelements (2) verhindert, solange die Kappe (1) in ihrer distalsten Position positioniert ist.

9. Einführvorrichtung nach einem der Ansprüche 1 bis 8, wobei die Kappe (1) und die Führungshülse (6) relativ zueinander derart vorgesehen sind, dass die Kappe (1) eine lineare Bewegung relativ zu der Führungshülse (6) ausführt, wobei die lineare Bewegung durch mindestens ein Paar von Führungsabschnitten (11, 62) geführt wird, wobei jedes Paar von Führungsabschnitten einen ersten Führungsabschnitt (11) in der Kappe (1) und einen zusammenwirkenden zweiten Führungsabschnitt (62) an der Führungshülse (6) aufweist, wobei die Einführvorrichtung optional mit mindestens drei solcher Paare (11a, 62a, 11b, 62b, 11c, 62c) von Führungsabschnitten versehen ist, wobei die Führungsabschnitte ferner optional im gleichen Abstand voneinander vorgesehen sind.

10. Einführvorrichtung nach einem der Ansprüche 1 bis 9, wobei die Kappe (1) mit einem oder mehreren Schiebeabschnitten (12) zum Schieben der Einführhülse (5) in Richtung des proximalen Endes (18) und optional mit Trennungsbegrenzungsmitteln (15, 16) zum Begrenzen einer Trennung der Kappe (1) von der Einführhülse (5) in Richtung des distalen Endes (17) versehen ist.

11. Einführvorrichtung nach einem der Ansprüche 1 bis 10, wobei die Kappe (1), die Einführhülse (5), der Retraktor (3), das Retraktorsteuerelement (2) und das elastische Element (4) gemeinsam mindestens entlang eines Abschnitts der Bewegung der Kappe (1) aus einer distalen Position in eine proximale Position bewegbar sind, optional ohne eine relative Bewegung relativ zueinander.

12. Einführvorrichtung nach einem der Ansprüche 1 bis 11, wobei das elastische Element (4) in einem vorgespannten Zustand vorgesehen ist, der zwischen dem Retraktor (3) und der Einführhülse (5) wirkt.

13. Einführsystem, umfassend eine Einführvorrichtung (1...6) nach einem der Ansprüche 1 bis 12 und eine medizinische Vorrichtung (7), die mit der Einführvorrichtung (1...6) einzuführen ist.

## Revendications

1. Dispositif d'insertion (1..6) pour l'insertion de manière au moins partielle d'un dispositif médical (7) comprenant un capteur d'analyte à travers la peau (9) d'un sujet, le dispositif d'insertion (1..6) ayant une extrémité proximale (18) destinée à être mise en contact avec la peau (9) et une extrémité distale (17) opposée à l'extrémité proximale (18), le dispositif d'insertion (1..6) comprenant :
un manchon de guidage (6) fourni au niveau de l'extrémité proximale (18) et destiné à être placé en contact avec la peau (9) pour un processus d'insertion ;
un capuchon (1) fourni au niveau d'une extrémité distale (17), sur lequel on peut appuyer pour réaliser au moins une partie du processus d'insertion tout en le déplaçant en direction de l'extrémité proximale (18) ;
un manchon d'insertion (5) fourni au moins partiellement à l'intérieur du manchon de guidage (6) et/ou du capuchon (1) et fourni avec un support (55) pour le dispositif médical (7) et le manchon d'insertion (5) étant mobile par rapport au manchon de guidage (6) ;
un rétracteur (3) conçu pour rétracter une partie (8) fournie avec le dispositif médical (7), le rétracteur (3) étant mobile le long d'une trajectoire de mouvement (28, 56) ;
un élément élastique (4) tel qu'un ressort, qui peut solliciter le rétracteur (3) vers l'extrémité distale (17) le long de la trajectoire de mouvement (28, 56) ; et
un élément de commande de rétracteur (2) qui commande le mouvement du rétracteur (3) en bloquant ou en libérant le mouvement du rétracteur (3) le long de la trajectoire de mouvement (28, 56) et qui peut lui-même être mobile, tel que mis en rotation, par rapport au rétracteur (3) ;
dans lequel le dispositif d'insertion est **caractérisé en ce que** l'élément de commande de rétracteur (2) est monté de manière coulissante en rotation sur une partie de manchon interne du manchon d'insertion (5).

2. Dispositif d'insertion selon la revendication 1, dans lequel l'élément de commande de rétracteur (2) peut être mis en rotation entre une position dans laquelle il bloque le mouvement du rétracteur (3) le long de la trajectoire de mouvement (28, 56) et une autre position dans laquelle il libère le mouvement du rétracteur (3) le long de la trajectoire de mouvement (28, 56).

3. Dispositif d'insertion selon la revendication 1 ou 2, dans lequel l'élément de commande de rétracteur (2) peut être mis en rotation par une partie (65) du manchon de guidage (6), qui entre en contact avec l'élément de commande de rétracteur (2), tel qu'avec une partie saillante (24) de l'élément de commande de rétracteur (2), lors d'un mouvement relatif de l'élément de commande de rétracteur (2) par rapport au manchon de guidage (6) tel qu'un mouvement de l'élément de commande de rétracteur en direction de l'extrémité proximale.

4. Dispositif d'insertion selon l'une quelconque des revendications 1 à 3, dans lequel le mouvement du rétracteur (3) le long de la trajectoire de mouvement (28, 56) est limité à un mouvement linéaire dans la direction de l'extrémité proximale (18) à l'extrémité distale (17) ou vice versa, la limitation au mouvement linéaire étant éventuellement fournie par au moins une paire de parties de guidage (33, 56), la paire de parties de guidage comprenant une première partie de guidage telle qu'un rail (33) et une seconde partie de guidage en coopération telle qu'un rail négatif (56), l'une des deux parties de guidage (33, 56) étant fournie au niveau du manchon d'insertion (5) et l'autre partie de guidage (33, 56) étant fournie au niveau du rétracteur (3), dans lequel éventuellement deux ou trois telles paires de parties de guidage sont fournies.

5. Dispositif d'insertion selon l'une quelconque des revendications 1 à 4, dans lequel l'élément de commande de rétracteur (2) est fourni avec au moins une partie de guidage (28) qui peut être amenée en alignement avec une autre partie de guidage (56) telle que la partie de guidage de la revendication 4 du manchon d'insertion (5) afin de libérer le mouvement du rétracteur (3) le long d'une partie de la trajectoire de mouvement et de bloquer le mouvement du rétracteur (3) le long de la partie de la trajectoire de mouvement (28, 56) dans le cas où la partie de guidage (28) de l'élément de commande de rétracteur (2) n'est pas dans un tel alignement.

6. Dispositif d'insertion selon l'une quelconque des revendications 1 à 5, dans lequel l'élément de commande de rétracteur (2) est fourni avec au moins une saillie de blocage (26) qui est fournie pour interagir avec une partie de blocage (19) du capuchon (1), le contact de la saillie de blocage (26) et de la partie de blocage (19) empêchant la rotation de l'élément de commande de rétracteur (2) dans une position dans laquelle il libère la trajectoire de mouvement (28) du rétracteur (3).

7. Dispositif d'insertion selon la revendication 6, dans lequel le contact de l'au moins une saillie de blocage (26) de l'élément de commande de rétracteur (2) et de la partie de blocage (19) du capuchon (1) est possible dans une position du capuchon (1) dans sa position la plus proximale lorsque l'on appuie dessus.

8. Dispositif d'insertion selon l'une quelconque des revendications 1 à 7, dans lequel l'élément de commande de rétracteur (2) comprend une partie de verrouillage (25) qui est en contact avec une partie de prise (13) du capuchon (1) qui empêche la rotation de l'élément de commande de rétracteur (2) tant que le capuchon (1) est positionné au niveau de sa position la plus distale.

9. Dispositif d'insertion selon l'une quelconque des revendications 1 à 8, dans lequel le capuchon (1) et le manchon de guidage (6) sont fournis relativement l'un par rapport à l'autre de telle sorte que le capuchon (1) réalise un mouvement linéaire par rapport au manchon de guidage (6), où le mouvement linéaire est guidé par au moins une paire de parties de guidage (11, 62), chaque paire de parties de guidage ayant une première partie de guidage (11) dans le capuchon (1) et une seconde partie de guidage (62) en coopération sur le manchon de guidage (6), dans lequel le dispositif d'insertion est éventuellement fourni avec au moins trois telles paires (11a, 62a, 11b, 62b, 11c, 62c) de parties de guidage, dans lequel les parties de guidage sont en outre éventuellement fournies espacées de manière équidistante les unes des autres.

10. Dispositif d'insertion selon l'une quelconque des revendications 1 à 9, dans lequel le capuchon (1) est fourni avec une ou plusieurs parties de poussée (12) pour pousser le manchon d'insertion (5) vers l'extrémité proximale (18) et éventuellement avec des moyens de limitation de séparation (15, 16) pour limiter une séparation du capuchon (1) loin du manchon d'insertion (5) vers l'extrémité distale (17).

11. Dispositif d'insertion selon l'une quelconque des revendications 1 à 10, dans lequel le capuchon (1), le manchon d'insertion (5), le rétracteur (3), l'élément de commande de rétracteur (2) et l'élément élastique (4) sont mobiles d'une position distale à une position proximale conjointement au moins le long d'une partie du mouvement du capuchon (1), éventuellement sans mouvement relatif les uns par rapport aux autres.

12. Dispositif d'insertion selon l'une quelconque des revendications 1 à 11, dans lequel l'élément élastique (4) est fourni dans un état préchargé agissant entre le rétracteur (3) et le manchon d'insertion (5).

13. Système d'insertion comprenant un dispositif d'insertion (1...6) selon l'une quelconque des revendications 1 à 12 et un dispositif médical (7) destiné à être inséré avec le dispositif d'insertion (1...6).
